# NEW EUROPEAN PATENT SPECIFICATION

(11) **EP 0 783 346 B2**
(45) Date of publication and mention of the opposition decision: **17.01.2007**
(45) Mention of the grant of the patent: 01.12.1999
(21) Application number: 95936233.6
(22) Date of filing: 29.09.1995
(51) Int. Cl.: A61N 1/30

(54) **IMPROVED IONTOPHORETIC DRUG DELIVERY DEVICE**
VERBESSERTE VORRICHTUNG ZUR IONTOPHORETISCHEN VERABREICHUNG VON MEDIKAMENTEN
SYSTEME AMELIORE POUR L'ADMINISTRATION D'UN MEDICAMENT PAR IONOPHORESE

(30) Priority: 30.09.1994 US 315378; 30.09.1994 US 315532; 30.09.1994 US 315533
(43) Date of publication of application: 16.07.1997
(73) Proprietor: Vyteris, Inc., Fair Lawn, New Jersey 07410-2700 (US)
(72) Inventor: FLOWER, Ronald, J., Vernon, NJ 07461 (US)
(74) Representative: Lawrence, Peter Robin Broughton
(86) International application number: PCT/US1995/012635
(87) International publication number: WO 1996/010442

(56) References cited:
- EP-A- 0 160 262
- EP-A- 0 308 572
- EP-A- 0 461 680
- EP-A- 0 497 473
- WO-A-92/04937
- US-A- 3 982 320
- US-A- 4 221 223
- US-A- 4 230 127
- US-A- 4 257 661
- US-A- 4 474 570
- US-A- 4 557 723
- US-A- 4 835 060
- US-A- 4 865 582
- US-A- 4 942 883

## Description

### BACKGROUND OF THE INVENTION

### Field of the Invention

The present invention relates generally to an iontophoretic delivery system tor transcutaneous drug delivery, and more specifically relates to an iontophoretic assembly including a drug-containing patch and a controller which establishes electrical connection therebetween to effect iontophoretic drug delivery.

### Description of the Prior Art

Iontophoresis may be defined as the non-invasive transdermal delivery of medicine/chemicals. This process has become an increasingly popular and effective method tor the delivery of pharmaceuticals. In practice, the process of iontophoretic drug delivery is typically achieved by placing a medicine (in ionic form) on a carrier, and attaching the medicine-containing carrier to a patient. A pair of electrodes are placed in contact with the patient's skin and in close proximity with the carrier. An electric current is provided by the electrodes through the skin. The electric current causes the ionic medicine to diffuse from the carrier of the patch through the skin.

Delivery of a drug to a patient iontophoretically may be best accomplished in a number of ways, such as at a constant rate over a long time period, or periodically at regular intervals. In order to ensure proper drug delivery, it is necessary for the drug-containing carrier to be maintained in contact with the patient's skin. The ion-tophoretic delivery system may include a drug-containing carrier such as an adhesive patch. The system may also include a source of electric power which is connectable to the patch for providing the proper electric current for transmission of the drug in accordance with the desired rate of delivery. Such a device is known from WO-A-9404937.

Proper iontophoretic delivery of the drug contained on the patch through the skin of the patient is dependent upon the level and duration of the current applied across the electrodes. It is desirable that a good clean electrical signal be impressed across the electrodes so that proper administration of the drug is achieved. Further, as electrical current is being passed directly through the skin of the patient, fluctuations in current applied between the electrodes may be felt by the patient. It is not uncommon tor the patient to experience slight discomfort if the current applied across the electrodes spikes or otherwise fluctuates. Such spikes or fluctuations can be caused by interference at the electrical interface between the patch and the controller. Dirt, debris or other contaminants lodged between the interconnecting surfaces may have a tendency to create electrical "noise" which can result in current variations. As described in US-4,557,723, an electrophoretic applicator can include means to maintain constant current flow during the period the medicament is being delivered.

The electrical connections between the patch and controller in known drug delivery devices typically utilize resiliently biased conventional mechanical contacts. With this type of connection, wear and corrosion from the repeated cycling of connection and removal of the patch with respect to the controller, and from environmental conditions, may have a deleterious effect on the contacts. The deleterious effects include the abrupt cessation of current delivery to the patch due to the "making and breaking" between the contacts. These current transients which may be produced may result in an uncomfortable sensation to the patient.

US 4,752,285 describes iontophoresis apparatus where wires running from a current source are attached to the apparatus and remote electrode.

In transdermal delivery devices where the controller is detachable from the patch, the materials utilized on the patch and controller for electrical coupling must be electro-chemically compatible with one another. Otherwise, electrolysis and corrosion may result affecting the quality of the electrical connection. This may result in electrical noise or current transients which may cause patient discomfort.

It is well known to construct mating electrical contacts of the conductive spring variety where the contacts are interconnected with such force that the mating deflectable spring portions wipe against each other thereby cleaning the interconnection. However, as may be appreciated, in an iontophoretic delivery system the electrical pads of the patch are formed on a thin flexible surface which is unable to withstand the forces normally seen in mating spring contacts. This is because the patch is designed for single use. Such forces may have a tendency to scrape away the conductive pads or otherwise damage the conductive portions of the patch. An attempt to reduce the interconnection force may result in losing the benefit achievable by sliding spring-deflectable contacts against one another.

It is therefore desirable to provide an interconnection assembly which permits the reliable, dean interconnection between a drug-containing flexible patch, positionable on the skin of a patient, and a source of electrical current tor permitting iontophoretic delivery of the drug.

### OBJECTS AND SUMMARY OF THE INVENTION

It is an object of the present invention to provide an improved iontophoretic drug delivery system for attachment to the skin of a patient for delivering a drug transcutaneously.

It is a further object of the present invention to provide an improved iontophoretic drug delivery system where the system includes a medicament-containing patch positionable on the skin of the patient and an electronic controller for controlling the iontophoretic delivery of the medicament through the skin.

It is still a further object of the present invention to provide a iontophoretic drug delivery system where the controller may be easily removably attached to the medicament-containing patch.

It is another object of the present invention to provide a drug delivery device which occupies minimal skin area on the patient and which is not cumbersome to the patient.

It is still another object of the present invention to provide an iontophoretic drug delivery device including an electrical connector which permits the reliable electrical interconnection between a drug delivery patch and a detachable controller.

It is a further object of the present invention to provide an iontophoretic drug delivery controller having an electrical connector which is not subject to electrolysis and/or corrosion.

It is still a further object of the present invention to provide improved electrical connection between conductive portions of a medicament-containing flexible patch and electrical contacts of a controller.

It is yet another object of the present invention to provide an iontophoretic drug delivery device having an electrical connector which overcomes the disadvantages of known electrical connectors of iontophoretic drug delivery devices.

The present invention provides an iontophoretic drug delivery system for delivering medication to an applied area of a patient, and which is defined in claim 1.

In accordance with one form of the present invention the apparatus includes a medicament containing disposable patch removably attachable to a patient's skin in combination with a controller which electrically controls the delivery of the medicament. The patch includes a flexible planar body having a first surface containing the medicament, an opposed second surface and an extending planar tab for insertion within a region of the controller. The first surface of the planar patch body is supportable on the skin of the patient The opposed second surface of the patch body has the controlier removably attached thereto. The patch is secured to the controller by inserting the tab within the controller. The tab of the patch body may include an opening therethrough. The housing may include a post which receives the tab opening to secure the tab within the controller housing.

The tab of the patch is received in a slot formed in a sidewall of the controller housing. The flexible patch is then bent about its tab and secured to the top surface of the controller by Velcro mating hook and loop fasteners respectively mounted on the top surfaces of the controller housing and patch. In this way, the controller may be conveniently mounted on top of the patch, as the patch rests against the patient's skin. The bottom of the patch containing the drug to be delivered is exposed and may be adhesively secured to the patient's skin. The tab of the patch body includes a plurality of spaced-apart electrical contacts that are coupled to at least first and second spaced-apart electrodes which, when a voltage is applied thereto, induces an electric current flow which transmits the medicament from the patch to the patient's skin.

The controller may include a power supply for providing a source of electric current and a circuit mounted on a printed circuit board for controlling the power supply. The controller also includes a connector which is positioned to engage the printed circuit board of the controller and the tab on the patch. The connector is of the elastomeric type. In one embodiment, the elastomeric connector includes a plurality of spaced-apart parallel conductive sections while the printed circuit board includes plurality of spaced-apart electrical contacts. Each of the contacts engages at least one of the plurality of conductive sections. The elastomeric connector is positioned such that when the tab of the patch body is inserted within the controller, the spaced-apart electrical contacts are respectively electrically coupled to at least one of the plurality of spaced-apart conductive sections of the elastomeric connector so that the at least first and second electrodes are electrically coupled to the power supply and printed circuit board of the controller.

### BRIEF DESCRIPTION OF THE DRAWINGS

Figure 1 is a perspective view of the iontophoretic drug delivery system of the present invention wherein a patch and controlier are in a disconnected configuration.
Figure 2 is a perspective view of the iontophoretic drug delivery system of the present invention wherein a patch and controller are in a connected configuration.
Figure 3 is a top plan view of a portion of the patch and controller combination of Figure 2.
Figure 4 is a cross-sectional view of the patch and controller combination of Figure 3 taken across line 4-4.
Figure 5 is a cross-sectional view of the patch and controller combination of Figure 4 taken along lines 5-5.
Figure 6 is a top perspective view of the iontophoretic drug delivery system of the present invention wherein the patch is supported on the skin of a patient and the controller is connected to the patch.
Figure 7 is a perspective view of an elastomeric connector utilized in the present invention electrically coupling spaced-apart electrical contacts of a printed circuit board envisioned to be used in a controller of a drug delivery device with spaced-apart electrical contacts of a patch envisioned to be used in such a device.
Figure 8 is a perspective view of the elastomeric connector utilized in an alternative embodiment of the present invention.

### DETAILED DESCRlPTION OF THE PREFERRED EMBODIMENTS

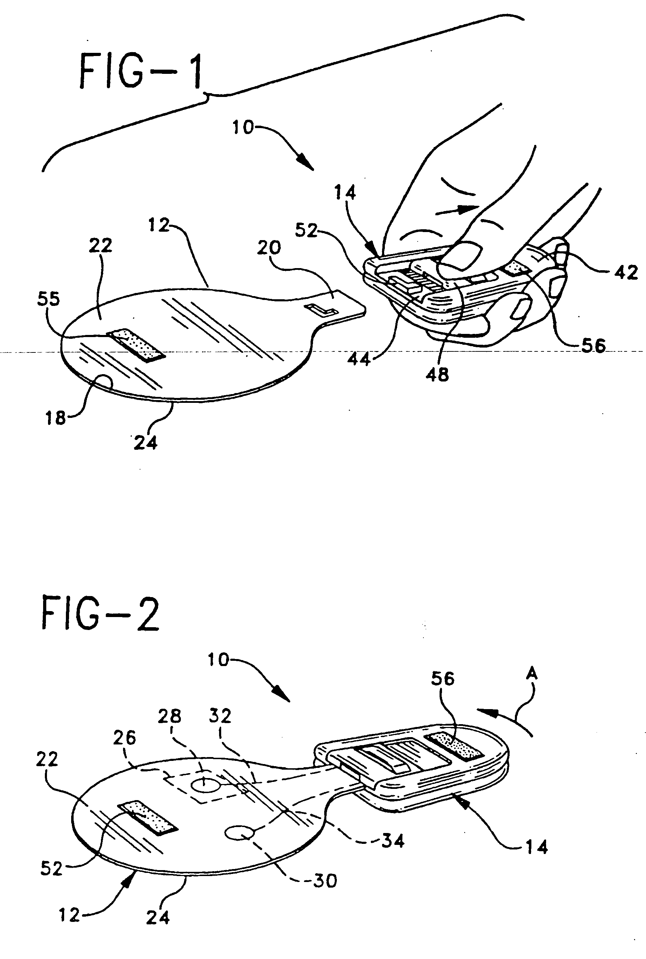

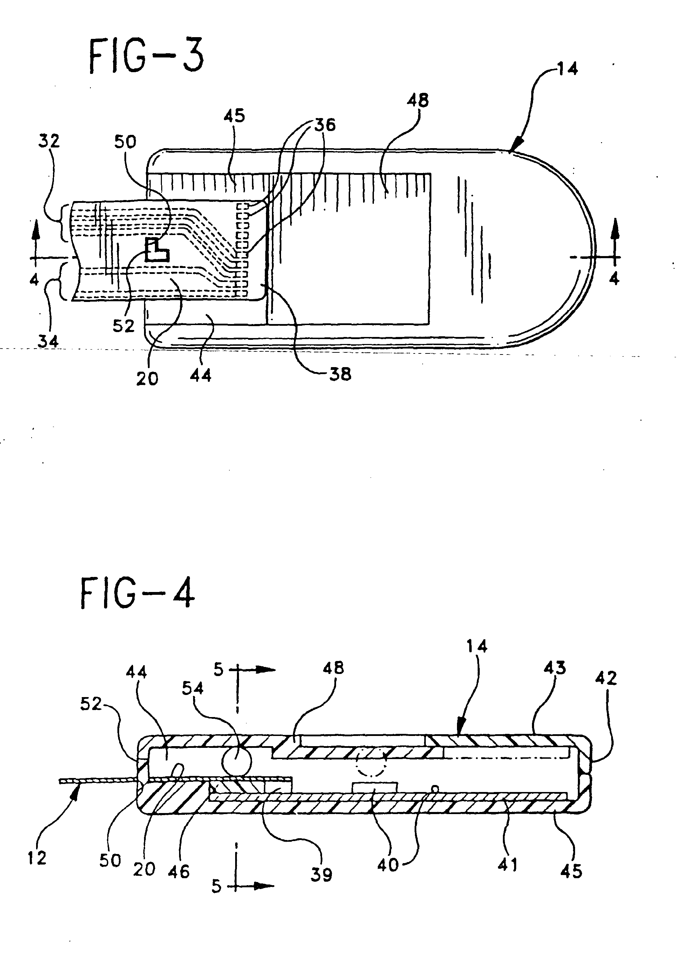

Referring initially to Figures 1 and 2 of the drawings, an iontophoretic drug delivery system 10 basically includes a patch 12 and a controller 14. Patch 12 is a generally planar flexible member formed of biocompatible material. Patch 12 may be formed of woven or non-woven textiles or polymers or any other material as is well known in the art Patch 12 preferably induces an adhesive (not shown) which permits the patch to be attached to the skin 16 of the patient (Fig. 6). In the preferred embodiment, patch 12 includes an enlarged substantially circular body 18 and an extending narrow tab 20. Patch body 18 includes opposed planar surfaces 22 and 24. Planar surface 24 is disposed for attachment to a patient's skin and includes a drug reservoir 26 which contains an ionic pharmaceutical which is typically in gel form. While reservoir 26 is shown in the Figures, any other technique may be employed which effectively stores an ionic medicament tor transmittal to the patient's skin.

Skin contacting surface 24 of the patch may further include at least a pair of spaced apart electrodes 28 and 30. Each of electrodes 28 and 30 are positioned to be in contact with the skin when the patch 12 is attached thereto. The electrodes 28 and 30 are positioned such that an electric current path is established between the electrodes 28 and 30 through the skin of the patient. Electrode 28 is also electrically coupled to reservoir 26 in a manner well-known in the iontophoretic drug delivery industry. A direct current source may be coupled to the electrodes 28 and 30 such that electrode 28, which is in contact with reservoir 26, assumes the same charge as the ionized drug contained therein. Under the influence of electrical current passing from electrode 28 to electrode 30 through the skin, the drug contained in reservoir 26 is transcutaneously transmitted to the patient.

Referring now to Figure 3, electric current is supplied to electrodes 28 and 30 via electrical conductors 32 and 34. Each of conductors 32 and 34 may include one or more conductive paths extending from electrodes 28 and 30 to electrical contacts 36 positioned on a marginal edge 38 of tab 20. As will be described in further detail hereinbelow, electrical contacts 36 are optimally positioned for electrical connection to the controller 14 and its source of electric current.

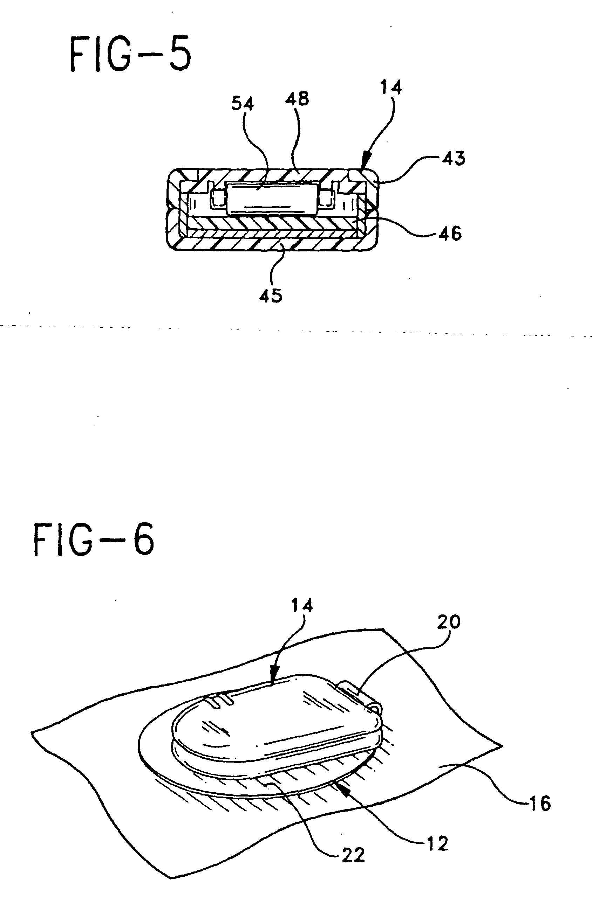

Referring now to Figure 4, controller 14 houses electronic components 40 which provide the controlled application of electric current to electrodes 28 and 30. As is known in the art, and in the preferred embodiment, the electrical components 40 include a source of electrical power such as a power supply 39. The electronic components and the power supply 39 may be mounted on a printed circuit board 41. The circuit of the controller is used to send a controlled electric current to electrodes 28 and 30.

Controller 14 includes a controller housing 42 having an upper wall 43. The controller also includes a cover 48 and a bottom wall 45. As shown in Figure 1, cover 48 may be manually moved to an open position exposing connection array 46 for electrical connection with electrical contacts 36 of tab 20. With cover 48 in an open position, tab 20 of patch 12 may be electrically coupled to controller 14 via connection array 46. Cover 48 may similarly be moved to a dosed position as shown in Figure 2, substantially covering electrical contacts 36 of tab 20 along with connection array 46.

In order to ensure accurate alignment of electrical contacts 36 with corresponding segments of connection array 46, tab 20 includes a keyed opening tc coincide to the structure of housing 42. Tab 20 includes an opening 50 which is designed to accept an upwardly extending post 52 of the housing. In a preferred embodiment, opening 50 and post 52 are generally rectangular in shape. However, any suitable configuration may be utilized. The controller also includes an open front end 44 which accommodates a suitable length of tab 20. Post 52 is centrally located adjacent connection array 46 so as to accommodate tab 20 and positionally confine tab 20 within housing 44. The key structure included on both opening 50 and post 52 prevents incorrect alignment of patch 12 with respect to controller 14. In the present embodiment, both opening 50 and post 52 have a generally L-shaped cross section, however, any other mating shape which would ensure correct alignment may be employed.

As mentioned above, housing 42 includes connection array 46 situated in electrical contact with printed circuit board 41. Connection array 46 may include a multiplicity of parallelly disposed, electrically conductive strips which are in electrical communication with the printed circuit board 41 and hence the components 40 mounted on the circuit board. The conductive strips of connection array 46 are also capable of being electrically coupled to the electrical contacts 36 of the patch when tab 20 is received by the opening 50 in the controller 14. In the present illustrative embodiment, printed circuit board 41 has a plurality of spaced-apart contacts 37 which the conductive strips of the array 46 engage.

As may be appreciated, suitable iontophoretic drug delivery of medicament contained in reservoir 26 to the skin of a patient is dependent upon both the amount and duration of current provided by electrodes 28 and 30. It is preferable that a good, dean electrical signal be provided across electrodes 28 and 30 so that proper transmission of the medicament is achieved. Further, as electric current is being passed directly through the skin of the patient, fluctuations in current flow between electrodes 28 and 30 may be felt by the patient. For example, the patient may encounter slight discomfort it the current applied across electrodes 28 and 30 spikes or otherwise fluctuates sharply. Such spikes or fluctuations can be caused by interference between the electrical connection of the connector array 46 and the patch contacts 36. The present invention : ensures that good clean electrical connection is established and maintained between the conductive portions of the patch and the conductive portions of the controllers, and also that there will not be any abrupt changes in the current due to variations in the impedance of the connection between the controller and the patch.

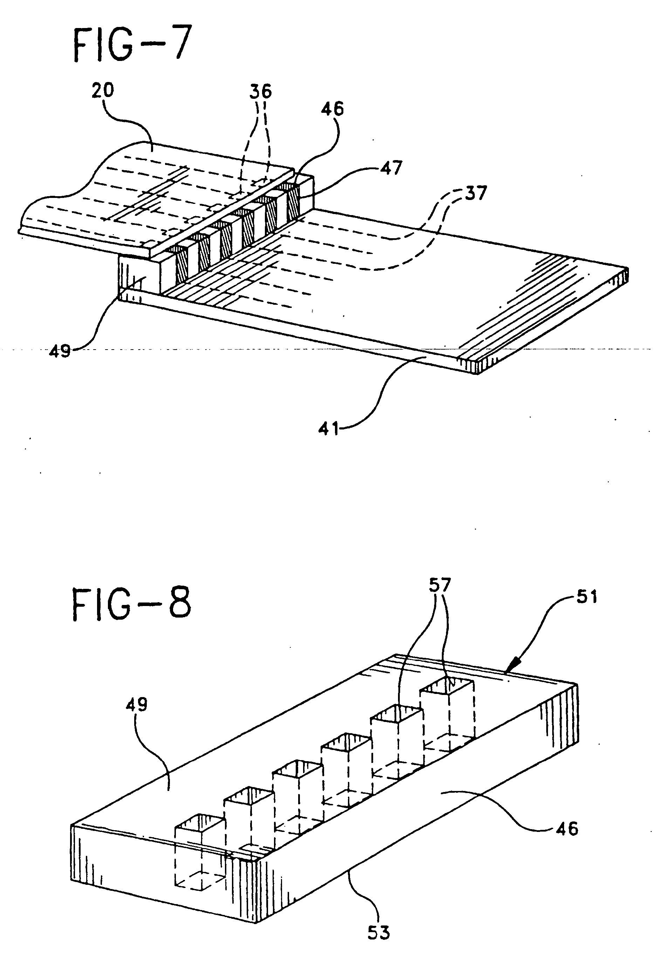

As shown in Figure 7, the connection array 46 includes an elastomeric connector electrically coupled to the power supply 39 and circuit board 41. The elastomeric connector is made of elastomeric material and is preferably a "zebra" strip, which has alternating sections of electrically conductive material 47 and electrically non-conductive material 49 as known in the art. The zebra strip is a commercially available product and a suitable zebra strip is manufactured by Technit Corporation. Typically, the non-conductive portion is silicone while the conductive portion is substantially carbon doped silicone. The conductive strips 47 may be spaced apart by only .002 centimeters(.005 inches), so that several conductive strips 47 may be in contact with the relatively wider contacts of the printed circuit board and patch to ensure a good connection between the patch and circuit board.

Referring again to Figure 7, the elastomeric connector is preferably securely affixed across the plurality of contacts 37 of the circuit board 41 for aiding in the electrical connection to the contacts. Preferably, the alternating strips of electrically conductive material 47 and electrically non-conductive 49 material are spaced apart so as to contact correspondingly spaced contacts 37 of the printed circuit board 41. As shown in Figures 2, 4, and 7, when the tab 20 of patch 12 is inserted within the controller, the plurality of spaced-apart electrical contacts 36 of the patch coincide with and electrically couple to the electrically conductive portions 47 of the elastomeric connector 46. Therefore, as shown in Figure 7, an electrical connection is achieved between the patch 12 and controller 14.

The zebra strip is preferred because it is capable of having a plurality of electrical paths which permit conduction of electric current in two directions, but prevent electrical conduction in a third substantially orthogonal direction. By permitting electrical connection along two axis, the zebra strip can easily adapt to the devices to which it will be coupled and provide coupling with devices located in different planes. In addition, negligible electrical noise is created with the elastomeric connector. Furthermore, the elastomeric connector is an inert material so that there is no junction potential and no electrolysis or corrosion of the connector. As a result, the resistance of the connection between the patch and the controller will not fluctuate. Moreover, the elastomeric connector is preferably made of a resilient material. Therefore, if pressure is applied to the connector, the connector will conform to the contour of the device to which it is coupled. This aids in providing a better electrical connection of the controller and patch. Finally, the elastomeric connector is non-abrasive and will not wear away the electrical contacts which are sometimes only painted on the surface of a device. This provides a better flow of current and no need to replace the connector or the devices to which it is attached because of wearing.

In an alternative form of the present invention and referring now to Figure 8, the elastomeric connector 46 consists of a section of electrically non-conductive (insulative) material 49 having first and second surfaces 51, 53. The connector may also include a plurality of electrically conductive elastomeric plugs 57 which extend through the non-conductive material 49 from the first surface 51 to the second surface 53. This type of elastomeric connector is typically specifically designed to coincide with the lay-out of the spaced-apart electrical contacts of the patch to which the controller is coupled. The conductive elastomeric plugs are typically positioned on the connector so as to provide good electrical contact with the patch electrical contacts. In a preferred embodiment, the plugs are specifically dimensioned and positioned within the elastomeric connector so that the conductive plugs will contact only one of the spaced apart electrical contacts of the patch. This type of connector is affixed to the printed circuit board 41 as previously described.

In order to ensure that adequate electrical coupling between electrical contacts 36 of tab 20 and the connection array 46 is maintained, cover 48 carries a captively retained pressure roller 54. As shown in Figure 4, the roller 54 is movable upon closure of cover 48 over electrical contacts 36 of tab 20 and connection array 46 to force electrical contacts 36 onto the conductive portions of connection array 46 establishing good electrical connection therebetween. The engagement of roller 54 also serves to secure patch 12 in connection with controller 14.

Referring again to Figure 2. patch 12 and controller 14 include attachment means so as to permit the releasable support of controller 14 on patch 12 after interconnection between electrical contacts 36 and connective array 46 is established. Surface 22, which is opposed to skin-engaging surface 24 of patch 12, and the upper surface of housing wall 43 include cooperating fastening elements 55 and 56. In the present illustrative embodiment, the cooperative fastening elements include conventional hook and loop fasteners of the type sold under the trademark VELCRO. One cooperating fastening element 55 is secured adhesively or otherwise to patch 12 on surface 22 while the other cooperating fastening member 56 is secured by adhesive or otherwise to the upper surface of wall 43 of housing 42. As will be described in further detail hereinbelow, attachment of the mating hook and loop fasteners 55 and 56 provides for removable support of controller 14 on patch 12.

Having described the components of the patch and controller assembly 10 of the present invention, its operation will now be described.

Patch 12 may be adhesively secured to the skin 16 of the patient. Surface 24 of patch 12 is placed in intimate contact with the skin 16 so that electrodes 28 and 30 as well as drug-containing reservoir 26 are supported in good intimate contact with the skin 16. In order to initiate transcutaneous iontophoretic drug delivery from reservoir 26. controller 14 is connected to patch 12. Cover 48 is retracted rearwardly opening front end 44 of controller 14. Housing 42 is slipped over extending tab 20 of patch 12 so that opening 50 in tab 20 is seated over upwardly extending post 52 of housing 42. Proper planar orientation is assured between patch 12 and controller 14 due to the key matability between opening 50 and post 52. Cover 48 is then slid forwardly, closing cover 48 over tab 20.

The captively retained roller 54 is provided over tab 20 forcing electrical contacts 36 into secure electrical engagement with the conductive portions of connection array 46. The conductive portions of connection array 46 are also electrically coupled to the spaced-apart conductive runners 37 of the printed circuit board 41 within the controller, Closure of cover 48 prevents removal of patch 12 from controller 14 as cover 48 overlies post 52. As controller 14 is designed to be left in electrical connection with patch 12 during drug delivery, controller 14 may be fastened to patch 12 so that it will be conveniently retained on the skin of the patient. As shown in Figure 2. once patch 12 is connected to controller 14, the controller may be flipped up in the direction of arrow A so that the mating hook and loop fasteners 55 and 56 engage each other to removably fasten controller 14 to patch 12 as show in Figure 6. The controller 14 is comfortably retained on the skin of the patient during iontophoretic drug delivery. When the particular drug delivery is complete, the controller may be removed by separating the mating hook and loop fasteners 55 and 56. Cover 40 may then be retracted exposing post 52 permitting removal of tab 20 from controller 14. The controller may be then placed aside until the next administration of the drug is needed. The patch 12 may remain on the skin of the patient, eliminating the need for frequent replacement of the patch.

## Claims

1. An iontophoretic drug delivery system for delivering medication to an applied area of a patient, which includes: an iontophoretic drug delivery patch (12) for placement against the skin of a patient, the iontophoretic drug delivery patch (12) including a medicament, and at least first and second electrodes (28, 30); a controller (14) mateable with the patch (12), the controller (14) being adapted to control current provided to the electrodes (28, 30) of the patch, the controller (14) having at least two controller contacts (37) **characterised in that** the patch (12) includes a plurality of spaced-apart electrical contacts (36), wherein at least a first electrical contact and a second electrical contact of the plurality of spaced-apart electrical contacts (36) are respectively electrically coupled to the first and second electrodes (28, 30), and **in that** the controller (14) includes an elastomeric connector (46) disposed between and electrically in contact with the at least two contacts (37) of the controller (14) and the first and second electrical contacts (36) of the patch (12) when the controller (14) is mated with the patch (12) and thereby establishing an electric current path from the controller, between the first and second electrodes through the skin of the patient, and back to the controller.

2. An iontophoretic drug delivery system according to claim 1, wherein the elastomeric connector (46) includes at least first and second electrically conductive sections (47) and a first electrically insulative section (49), wherein the first electrically insulative section (49) prevents current flow between the first conductive section to the second conductive section.

3. An iontophoretic drug delivery system according to claim 1, wherein the elastomeric connector (46) is of an electrically insulative material (49) having first and second surfaces (51, 53), and includes a plurality of conductive sections (57) therein, each of the plurality of conductive sections extending from the first surface (51) to the second surface (53).

4. An iontophoretic drug delivery system according to claim 3, wherein the plurality of conductive sections (57) are disposed within the electrically insulative material (49) so as to electrically couple at least one of the plurality of conductive sections (47) with at least one of the plurality of spaced-apart electrical contacts (36) of the iontophoretic drug delivery patch (12) when the controller (14) is mated with the patch (12).

5. An iontophoretic drug delivery system according to claim 1, wherein the elastomeric connector (46) includes a plurality of spaced-apart transverse conductive sections (47), wherein at least one of the plurality of conductive sections engages at least one of the plurality of spaced apart electrical contacts (36) of the iontophoretic drug delivery patch (12) so as to electrically couple the corresponding spaced-apart electrical contacts of the patch (12) to the controller (14).

6. An iontophoretic drug delivery system according to claim 5, wherein the controller (14) includes a printed circuit board (41) having a plurality of the controller contacts (37).

7. An iontophoretic drug delivery system according to any preceding claim, wherein the iontophoretic drug delivery patch (12) has a medicament containing portion (26).

8. An iontophoretic drug delivery system according to claim 1, wherein the medicament-containing disposable patch (12) is removably positionable on the skin of a patient for permitting iontophoretic delivery of medicament transcutaneously and the controller (14) includes electronic components (40) for electrically controlling the medicament delivery, and the patch (12) includes a flexible planar patch body (18) having a medicament-containing first surface (24), an opposed second surface (22), the first surface of the planar patch body being supportable on the skin of the patient; and wherein the patch (12) includes an extending planar tab (20) for insertable electrical accommodation in the controller (14), and wherein the opposed second surface of the patch body and the controller (14) include a co-operative removable fastening means (55, 56) for removably fastening the controller (14) to the patch (12) and for maintaining the controller (14) in a fastened condition with respect to the patch (12) with the tab electrically accommodated in the controller.

9. An iontophoretic drug delivery system according to claim 8, wherein the controller (14) includes a controller housing (42) having a first housing surface (44) and an opposed second housing surface (48), wherein upon insertion of the tab (20) into the housing (42) the first housing surface (44) is generally aligned with the second surface (22) of planar patch body (18).

10. An iontophoretic drug delivery system according to claim 9, wherein the co-operative removable fastening means (55, 56) includes a first fastening element (56) supported on the first surface of the housing (42) and a second fastening element (55) supported on the second surface of the planar body (18), and wherein the first and second fastening elements are securable to each other upon placement of the first housing surface (44) in contact with the second surface (22) of the planar patch body (18).

11. An iontophoretic drug delivery system according to claim 10, wherein the housing (56) includes a housing post (52) extending adjacent the housing opening (44), wherein the tab (20) includes a tab opening (52) for insertably accommodating the post (52) therein upon insertion of tab in the housing, and wherein the housing (56) includes a movable cover (40) being movable between an open position exposing the post for removable accommodation of the tab and a closed position preventing removal of the tab from the post, and wherein the movable cover (40) includes a roller (54) for movement over the tab supported over the post.

## Patentansprüche

1. System zur iontophoretischen Verabreichung von Medikamenten für die Verabreichung einer Medikation auf einen Anwendungsbereich eines Patienten, das umfasst: ein iontophoretisches Verabreichungspflaster für Medikamente (12) zur Anbringung auf die Haut eines Patienten, wobei das iontophoretische Verabreichungspflaster für Medikamente (12) ein Medikament und mindestens eine erste und zweite Elektrode (28, 30) umfasst; einen Regler (14), der mit dem Pflaster (12) in Eingriff gebracht werden kann, wobei der Regler (14) so ausgeführt ist, dass er den Strom steuert, der den Elektroden (28, 30) des Pflasters zugeführt wird, wobei der Regler (14) mindestens zwei Reglerkontakte (37) aufweist, **dadurch gekennzeichnet, dass** das Pflaster (12) eine Vielzahl von beabstandeten elektrischen Kontakten (36) umfasst, bei dem mindestens ein erster elektrischer Kontakt und ein zweiter elektrischer Kontakt der Vielzahl von beabstandeten elektrischen Kontakten (36) entsprechend mit den ersten und zweiten Elektroden (28, 30) elektrisch gekoppelt sind, und **dadurch**, dass der Regler (14) ein elastomeres Verbindungselement (46) umfasst, das zwischen den und elektrisch in Kontakt mit den mindestens zwei Kontakten (37) des Reglers (14) und den ersten und zweiten elektrischen Kontakten (36) des Pflasters (12) angeordnet ist, wenn der Regler (14) mit dem Pflaster (12) in Eingriff gebracht ist und **dadurch** eine elektrische Strombahn vom Regler, zwischen der ersten und zweiten Elektrode durch die Haut des Patienten und zurück zum Regler bereitgestellt wird.

2. System zur iontophoretischen Verabreichung von Medikamenten nach Anspruch 1, bei dem das elastomere Verbindungselement (46) mindestens einen ersten und zweiten elektrisch leitenden Abschnitt (47) und einen ersten elektrisch isolierenden Abschnitt (49) umfasst, wobei der erste elektrisch isolierende Abschnitt (49) den Stromfluss zwischen dem ersten leitenden Abschnitt und dem zweiten leitenden Abschnitt verhindert.

3. System zur iontophoretischen Verabreichung von Medikamenten nach Anspruch 1, bei dem das elastomere Verbindungselement (46) aus einem elektrisch isolierenden Material (49) mit einer ersten und zweiten Fläche (51, 53) ist und darin eine Vielzahl von leitenden Abschnitten (57) umfasst, wobei sich jeder der Vielzahl von leitenden Abschnitten von der ersten Fläche (51) zur zweiten Fläche (53) erstreckt.

4. System zur iontophoretischen Verabreichung von Medikamenten nach Anspruch 3, bei dem die Vielzahl der leitenden Abschnitte (57) innerhalb des elektrisch isolierenden Materials (49) so angeordnet ist, dass mindestens einer der Vielzahl von leitenden Abschnitten (47) mit mindestens einem der Vielzahl von beabstandeten elektrischen Kontakten (36) des iontophoretischen Verabreichungspflasters für Medikamente (12) elektrisch gekoppelt wird, wenn der Regler (14) mit dem Pflaster (12) in Eingriff gebracht ist.

5. System zur iontophoretischen Verabreichung von Medikamenten nach Anspruch 1, bei dem das elastomere Verbindungselement (46) eine Vielzahl von beabstandeten leitenden Querabschnitten (47) umfasst, wobei mindestens einer der Vielzahl von leitenden Abschnitten mit mindestens einem der Vielzahl von beabstandeten elektrischen Kontakten (36) des iontophoretischen Verabreichungspflasters für Medikamente (12) so in Eingriff kommt, dass die entsprechenden beabstandeten elektrischen Kontakte des Pflasters (12) mit dem Regler (14) elektrisch gekoppelt werden.

6. System zur iontophoretischen Verabreichung von Medikamenten nach Anspruch 5, bei dem der Regler (14) eine Leiterplatte (41) mit einer Vielzahl von Reglerkontakten (37) beinhaltet.

7. System zur iontophoretischen Verabreichung von Medikamenten nach irgendeinem vorhergehenden Anspruch, bei dem das iontophoretische Verabreichungspflaster für Medikamente (12) einen Medikament enthaltenden Abschnitt (26) aufweist.

8. System zur iontophoretischen Verabreichung von Medikamenten nach Anspruch 1, bei dem das wegwerfbare Medikament enthaltende Pflaster (12) lösbar auf der Haut eines Patienten angeordnet werden kann, um die iontophoretische Verabreichung des Medikamentes transkutan zu gestatten, und der Regler (14) elektronische Bauelemente (40) für das elektrische Steuern der Verabreichung des Medikamentes umfasst, und das Pflaster (12) einen flexiblen planaren Pflasterkörper (18) mit einer Medikament enthaltenden ersten Fläche (24), einer gegenüberliegenden zweiten Fläche (22) umfasst, wobei die erste Fläche des planaren Pflasterkörpers auf der Haut des Patienten getragen werden kann; und bei dem das Pflaster (12) eine sich erstreckende planare Lasche (20) für das einsetzbare elektrische Aufnehmen im Regler (14) umfasst, und bei dem die gegenüberliegende zweite Fläche des Pflasterkörpers und der Regler (14) eine zusammenwirkende lösbare Befestigungseinrichtung (55, 56) für das lösbare Befestigen des Reglers (14) am Pflaster (12) und für das Beibehalten des Reglers (14) in einem befestigten Zustand bezüglich des Pflasters (12) umfassen, wobei die Lasche im Regler elektrisch aufgenommen wird.

9. System zur iontophoretischen Verabreichung von Medikamenten nach Anspruch 8, bei dem der Regler (14) eine Reglergehäuse (42) mit einer ersten Gehäusefläche (44) und einer gegenüberliegenden zweiten Gehäusefläche (48) umfasst, bei dem beim Einsetzen der Lasche (20) in das Gehäuse (42) die erste Gehäusefläche (44) im allgemeinen mit der zweiten Fläche (22) des planaren Pflasterkörpers (18) ausgerichtet ist.

10. System zur iontophoretischen Verabreichung von Medikamenten nach Anspruch 9, bei dem die zusammenwirkende lösbare Befestigungseinrichtung (55, 56) ein erstes Befestigungselement (56), das auf der ersten Fläche des Gehäuses (42) getragen wird, und ein zweites Befestigungselement (55), das auf der zweiten Fläche des planaren Körpers (18) getragen wird, umfasst und bei dem das erste und zweite Befestigungselement beim Anordnen der ersten Gehäusefläche (44) in Kontakt mit der zweiten Fläche (22) des planaren Pflasterkörpers (18) aneinander gesichert werden können.

11. System zur iontophoretischen Verabreichung von Medikamenten nach Anspruch 10, bei dem das Gehäuse (56) einen Gehäusevorsprung (52) umfasst, der sich angrenzend an die Gehäuseöffnung (44) erstreckt, bei dem die Lasche (20) eine Laschenöffnung (52) für das einsetzbare Aufnehmen des Vorsprunges (52) darin beim Einsetzen der Lasche in das Gehäuse umfasst, und bei dem das Gehäuse (56) eine bewegliche Abdeckung (40) umfasst, die zwischen einer offenen Position, die den Vorsprung für das lösbare Aufnehmen der Lasche freilegt, und einer geschlossenen Position, die das Entfernen der Lasche vom Vorsprung verhindert, beweglich ist und bei dem die bewegliche Abdeckung (40) eine Rolle (54) für die Bewegung über die Lasche umfasst, die über dem Vorsprung gehalten wird.

## Revendications

1. Système d'administration d'un médicament par ionophorèse pour administrer un médicament à une zone d'application d'un patient, englobant : un timbre d'administration du médicament par ionophorèse (12) destiné à être appliqué sur la peau d'un patient, le timbre d'administration du médicament par ionophorèse (12) englobant un médicament, et au moins des première et deuxième électrodes (28, 30); un dispositif de commande (14) pouvant être accouplé au timbre (12), le dispositif de commande (14) étant destiné à assurer la commande du courant transmis aux électrodes (28, 30) du timbre, le dispositif de commande (14) comportant au moins deux contacts de commande (37), **caractérisé en ce que** le timbre (12) englobe plusieurs contacts électriques espacés (36), au moins un premier contact électrique et un deuxième contact électrique des plusieurs contacts électriques espacés (36) étant respectivement accouplés électriquement aux première et deuxième électrodes (28, 30), et **en ce que** le dispositif de commande (14) englobe un connecteur élastomère (46) agencé entre les deux ou plus de deux contacts (37) du dispositif de commande (14) et les premier et deuxième contacts électriques (36) du timbre (12) et en contact électrique avec ceux-ci lors de l'accouplement du dispositif de commande (14) et du timbre (12) et établissant ainsi un trajet de courant électrique partant du dispositif de commande, passant entre les première et deuxième électrodes à travers la peau du patient, et revenant au dispositif de commande.

2. Système d'administration d'un médicament par ionophorèse selon la revendication 1, dans lequel le conducteur élastomère (46) englobe au moins des première et deuxièmes sections électroconductrices (47) et une première section à isolation électrique (49), la première section à isolation électrique (49) empêchant l'écoulement de courant de la première section conductrice vers la deuxième section conductrice.

3. Système d'administration d'un médicament par ionophorèse selon la revendication 1, dans lequel le connecteur élastomère (46) est composé d'un matériau à isolation électrique (49) comportant des première et deuxième surfaces (51, 53) et englobe plusieurs sections conductrices (57), chacune des plusieurs sections conductrices s'étendant à partir de la première surface (51) vers la deuxième surface (53).

4. Système d'administration d'un médicament par ionophorèse selon la revendication 3, dans lequel les plusieurs sections conductrices (57) sont agencées dans le matériau à isolation électrique (49) de sorte à accoupler électriquement au moins une des plusieurs sections conductrices (47) à au moins un des plusieurs contacts électriques espacés (36) du timbre d'administration du médicament par ionophorèse (12) lors de l'accouplement du dispositif de commande (14) et du timbre (12).

5. Système d'administration d'un médicament par ionophorèse selon la revendications 1, dans lequel le connecteur élastomère (46) englobe plusieurs sections conductrices transversales espacées (47), au moins une des plusieurs sections conductrices s'engageant dans au moins un des plusieurs contacts électriques espacés (36) du timbre d'administration du médicament par ionophorèse (12), de sorte à accoupler électriquement les contacts électriques espacés correspondants du timbre (12) au dispositif de commande (14).

6. Système d'administration d'un médicament par ionophorèse selon la revendication 5, dans lequel le dispositif de commande (14) englobe une plaquette de circuits imprimés (41) comportant plusieurs contacts du dispositif de commande (37).

7. Système d'administration d'un médicament par ionophorèse selon l'une quelconque des revendications précédentes, dans lequel le timbre d'administration du médicament par ionophorèse (12) comporte une partie contenant un médicament (26).

8. Système d'administration d'un médicament par ionophorèse selon la revendication 1, dans lequel le timbre jetable contenant le médicament (12) peut être positionné de façon amovible sur la peau d'un patient pour permettre l'administration transcutanée par ionophorèse du médicament, le dispositif de commande (14) englobant des composants électroniques (40) pour assurer la commande électrique de l'administration du médicament, le timbre (12) englobant un corps de timbre plat et flexible (18) comportant une première surface contenant un médicament (24), une deuxième surface opposée (22), la première surface du corps plat du timbre pouvant être supportée sur la peau du patient ; le timbre (12) englobant une patte plate à extension (20) en vue de la réception électrique par insertion dans le dispositif de commande (14), la deuxième surface opposée du corps du timbre et du dispositif de commande (14) englobant un moyen de fixation amovible de coopération (55, 56) pour fixer de façon amovible le dispositif de commande (14) au timbre (12) et pour maintenir la fixation du dispositif de commande (14) au timbre (12), la patte étant reçue électriquement dans le dispositif de commande.

9. Système d'administration d'un médicament par ionophorèse selon la revendication 8, dans lequel le dispositif de commande (14) englobe un boîtier du dispositif de commande (42) comportant une première surface de boîtier (44) et une deuxième surface de boîtier opposée (48), la première surface du boîtier (44) étant généralement alignée avec la deuxième surface (22) du corps plat du timbre (18) lors de l'insertion de la patte (20) dans le boîtier (42).

10. Système d'administration d'un médicament par ionophorèse selon la revendication 9, dans lequel le moyen de fixation amovible de coopération (55, 56) englobe un premier élément de fixation (56) supporté sur la première surface du boîtier (42) et un deuxième élément de fixation (55) supporté sur la deuxième surface du corps plat (18), les premier et deuxième éléments de fixation pouvant être fixés l'un à l'autre lors de la mise en contact de la première surface du boîtier (44) avec la deuxième surface (22) du corps plat du timbre (18).

11. Système d'administration d'un médicament par ionophorèse selon la revendication 10, dans lequel le boîtier (56) englobe un montant de boîtier (52) s'étendant près de l'ouverture du boîtier (44), la patte (20) englobant une ouverture de patte (52) en vue de la réception par insertion du montant (52) lors de l'insertion de la patte dans le boîtier, le boîtier (56) englobant un couvercle mobile (40), pouvant être déplacé entre une position ouverte exposant le montant en vue d'une réception amovible de la patte, et une position fermée, empêchant l'enlèvement de la patte du montant, le couvercle mobile (40) englobant un rouleau (54) destiné à être déplacé au-dessus de la patte supportée au-dessus du montant.
